(19) **Europäisches Patentamt / European Patent Office / Office européen des brevets**

(11) **EP 3 115 770 B1**

(12) **FASCICULE DE BREVET EUROPEEN**

(45) Date de publication et mention de la délivrance du brevet:
**13.04.2022 Bulletin 2022/15**

(21) Numéro de dépôt: **16178062.2**

(22) Date de dépôt: **05.07.2016**

(51) Classification Internationale des Brevets (IPC):
**G01N 21/78** *(2006.01)*  **G01N 33/49** *(2006.01)*
**G01N 21/77** *(2006.01)*

(52) Classification Coopérative des Brevets (CPC):
**G01N 21/78; G01N 33/49;** G01N 2021/7773;
G01N 2021/7783

(54) **PROCÉDÉ D'ESTIMATION D'UNE QUANTITÉ D'ANALYTE DANS UN LIQUIDE**

SCHÄTZUNGSVERFAHREN DER MENGE VON ANALYTEN IN EINER FLÜSSIGKEIT

METHOD FOR ESTIMATING AN AMOUNT OF ANALYTE IN A LIQUID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.07.2015 FR 1556445**

(43) Date de publication de la demande:
**11.01.2017 Bulletin 2017/02**

(73) Titulaires:
- **Commissariat à l'énergie atomique et aux énergies alternatives
  75015 Paris (FR)**
- **Avalun
  38054 Grenoble (FR)**

(72) Inventeurs:
- **COUTARD, Jean-Guillaume
  38660 SAINT-PANCRASSE (FR)**
- **POUTEAU, Patrick
  38240 MEYLAN (FR)**
- **CUBIZOLLES, Myriam-Laure
  CORENC 38700 (FR)**

(74) Mandataire: **INNOV-GROUP
310, avenue Berthelot
69372 Lyon Cedex 08 (FR)**

(56) Documents cités:
WO-A1-2013/071301    WO-A1-2014/068003
US-A1- 2013 126 712    US-A1- 2015 160 244

- DEMITRI NEVINE ET AL: "Detection of faulty glucose measurements using texture analysis", 2014 22ND EUROPEAN SIGNAL PROCESSING CONFERENCE (EUSIPCO), EURASIP, 1 septembre 2014 (2014-09-01), pages 2480-2484, XP032681886,

**Description**

## DOMAINE TECHNIQUE

Le domaine technique de l'invention est l'analyse de liquides corporels, en particulier du sang, afin de déterminer une quantité d'un analyte, et cela par une méthode optique. Une application est la mesure de la glycémie dans le sang.

## ART ANTERIEUR

La mesure de la glycémie est une mesure couramment pratiquée à l'aide de dispositifs de mesures portables, utilisables à domicile, dans des applications dénommées « Point of Care », terme anglais signifiant au chevet du patient, ou chez le patient.

La mesure de la glycémie peut être réalisée par une méthode optique. C'est par exemple le cas du dispositif décrit dans le brevet US5866349. Dans ce brevet, on décrit une méthode optique pour déterminer la concentration de glucose dans du sang total. Après une étape d'hémolyse, la méthode met en œuvre une réaction enzymatique entraînant la formation d'un indicateur coloré à partir d'un sel de tétrazolium.

Un photodétecteur mesure l'intensité d'un rayonnement lumineux transmis par un échantillon sanguin, ce dernier étant successivement illuminé par deux diodes électroluminescentes. Une première diode électroluminescente émet un premier faisceau lumineux dans une longueur d'onde de 660 nm, cette longueur d'onde étant comprise dans la bande spectrale d'absorption de l'indicateur coloré. Une deuxième diode électroluminescente émet un deuxième faisceau lumineux dans une longueur d'onde comprise entre 740nm et 940 nm, bande spectrale dans laquelle le sang présente une transmission élevée. Une comparaison de l'intensité transmise, à ces deux longueurs d'onde, permet d'estimer une quantité d'indicateur coloré formée dans l'échantillon, à partir de laquelle une concentration de glucose peut être déterminée.

Mais cette méthode suppose le recours à l'illumination successive de l'échantillon sanguin analysé par deux sources de lumière différentes. Il est donc nécessaire de disposer deux sources de lumière dans le dispositif d'analyse.

D'autre part, le brevet EP1875203 décrit un dispositif permettant d'estimer une quantité de glucose dans un échantillon sanguin, sans mettre en œuvre une étape d'hémolyse. Le principe de mesure repose également sur la formation d'un indicateur coloré issu de la réduction d'un sel de tétrazolium. L'échantillon sanguin est couplé au photodétecteur par deux lentilles, disposées successivement entre l'échantillon et le photodétecteur. Ces lentilles permettent d'augmenter le signal collecté par le photodétecteur. Le photodétecteur peut être un photodétecteur matriciel, de type CCD. De même que dans le brevet US5866349, deux sources de lumière sont utilisées, l'une émettant dans une bande spectrale d'absorption de l'indicateur coloré, l'autre émettant dans le proche infrarouge. La détection du rayonnement lumineux transmis par l'échantillon, illuminé dans l'infrarouge, permet d'estimer un taux d'hématocrite ; la détection du rayonnement lumineux transmis par l'échantillon dans la bande spectrale d'absorption de l'indicateur permet d'estimer une quantité de glucose, cette estimation étant corrigée du taux d'hématocrite préalablement déterminé. Mais la mise en œuvre d'un système optique complexe, basé sur deux lentilles hémisphériques, nuit à la compacité du dispositif, ainsi qu'à son coût.

La demande de brevet US2015/0160244 décrit un procédé pour quantifier la quantité d'un analyte, tel le glucose, dans un échantillon, en se basant sur l'intensité de pixels d'une image de l'échantillon. La demande de brevet US2013/0126712 décrit un procédé pour détecter un analyte, tel le glucose, dans un échantillon liquide.

La présente invention est une méthode simple et fiable, permettant d'obtenir une quantification de glucose, ou autre analyte, par le biais d'une réaction enzymatique aboutissant à la formation d'un indicateur coloré. Cette méthode ne nécessite pas l'illumination de l'échantillon par deux faisceaux lumineux dans deux bandes spectrales différentes. De plus, elle peut être mise en œuvre à l'aide d'un dispositif optique simple et compact.

## EXPOSE DE L'INVENTION

Un objet de l'invention est un procédé d'estimation d'une quantité d'un analyte dans un échantillon liquide, le procédé comportant les étapes suivantes :

> a) mélange dudit échantillon avec un premier réactif apte à former un indicateur coloré en présence dudit analyte dans l'échantillon,
> b) suite à ce mélange, illumination dudit échantillon à l'aide d'une source de lumière, apte à émettre un rayonnement lumineux vers l'échantillon,
> c) acquisition, à l'aide d'un photodétecteur matriciel, d'une image d'un rayonnement lumineux transmis ou réfléchi par l'échantillon,
> d) estimation d'une quantité dudit analyte en fonction de ladite image.

Le procédé peut comprendre les caractéristiques énumérées ci-dessous, prises isolément ou selon les combinaisons techniquement envisageables :

- L'étape d) peut comporter les sous-étapes suivantes :

> i) sélection d'une zone de mesure, dans ladite image, ladite zone de mesure comprenant une pluralité de pixels,
> ii) détermination, à l'intérieur de la zone de mesure, d'une région d'intérêt et d'au moins une région d'exclusion,

iii) estimation de ladite quantité d'analyte à partir d'une grandeur représentative de l'intensité des pixels dans ladite région d'intérêt, sans prise en compte de l'intensité des pixels de chaque région d'exclusion.

Cela permet de ne pas prendre en compte, lors de l'étape d), des parties de l'image non représentatives de la formation de l'indicateur coloré. Par ailleurs, le fait d'effectuer une estimation sur la base d'une image permet de tenir compte d'éventuelles variations spatiales de la quantité d'analyte ou de la formation d'indicateur coloré.

- La sous-étape ii) peut comprendre la détermination d'au moins une région d'exclusion, ladite région d'intérêt correspondant à une partie de ladite zone de mesure ne comportant pas chaque région d'exclusion ainsi déterminée.
  En particulier, la région d'intérêt est, dans la zone de mesure, complémentaire de chaque région d'exclusion déterminée. Cela permet d'estimer la quantité d'analyte sur l'ensemble de la zone de mesure, à l'exception de la région d'exclusion ou des régions d'exclusion déterminées. On maximise alors la surface de la région d'intérêt sur la base de laquelle l'estimation est réalisée.
  La région d'exclusion peut être déterminée par seuillage de l'image, ou par segmentation de ladite image.
  La sous-étape ii) peut notamment comprendre la détermination d'une pluralité de régions d'exclusion, distinctes et distantes les unes des autres, la région d'intérêt s'étendant entre ces régions d'exclusion. Chaque région d'exclusion peut en particulier être délimitée par un contour fermé, annulaire ou polygonal.
- L'échantillon comportant des particules, par exemple des globules rouges, le procédé peut comprendre un mélange de l'échantillon avec un deuxième réactif, dit réactif de lyse, apte à lyser lesdites particules. Ce mélange est notamment réalisé préalablement aux étapes c) et d), et par exemple lors de l'étape a). Chaque région d'exclusion peut alors correspondre à la trace d'une bulle d'air, ladite bulle d'air étant formée suite à la lyse desdites particules. Ainsi, l'estimation de la quantité d'analyte ne prend pas en compte la présence de ces bulles d'air.
- Le procédé peut comporter, suite au mélange de l'échantillon avec ledit deuxième réactif, et lors de l'étape d),

  la détermination d'un indicateur de lyse à partir de l'image acquise lors de l'étape c) ;
  l'estimation de la quantité d'analyte étant réalisée lorsque l'indicateur de lyse satisfait à un critère de lyse prédéterminé.

Le procédé peut être tel que lorsque l'indicateur de lyse ne satisfait pas audit critère de lyse, l'étape c) est réitérée, de telle sorte qu'une pluralité d'indicateurs de lyse sont déterminés, à partir d'images acquises à différents instants. Chaque indicateur de lyse peut notamment être déterminé en formant une image de comparaison, représentant une comparaison entre deux images acquises à différents instants $(t_i, t_{i-1})$ en particulier sous la forme d'une différence. L'indicateur de lyse peut alors être obtenu par une grandeur statistique de chaque image, ou de chaque image de comparaison, la grandeur statistique étant par exemple une moyenne, une médiane ou un facteur de dispersion de type variance ou écart-type. Cela permet de suivre l'évolution de la lyse à l'aide de l'image, et de détecter l'instant à partir duquel l'estimation de la quantité d'analyte peut être effectuée.

- Le procédé peut comprendre une étape d'introduction de l'échantillon dans une chambre fluidique, les étapes c) et d) étant effectuées à un instant prédéterminé après ladite introduction.
- Le procédé peut comprendre la formation d'une pluralité d'images successives du rayonnement lumineux transmis ou réfléchi par l'échantillon, la quantité d'analyte étant déterminée, lors de l'étape d), selon l'évolution de l'intensité de chaque image en fonction du temps.
- Lors de l'étape d), la grandeur représentative de l'intensité des pixels dans ladite région d'intérêt peut comporter l'intégrale ou la moyenne des pixels dans ladite région d'intérêt.
- Le photodétecteur peut être situé à une distance de l'échantillon inférieure à 1 cm, ce qui permet la mise en oeuvre du procédé par un dispositif compact.
- Selon un mode de réalisation, il n'y a aucune optique de grossissement entre l'échantillon et le photodétecteur, ce qui permet la mise en oeuvre du procédé par un dispositif compact et peu onéreux.
- L'analyte peut être le glucose.
- L'échantillon comporte un liquide corporel, par exemple du sang.
- L'échantillon peut être disposé entre ladite source de lumière et le photodétecteur, de telle sorte que le photodétecteur détecte un rayonnement transmis par l'échantillon, l'image acquise par le photodétecteur étant alors une image dite de transmission.
- L'échantillon peut être disposé face à la source de lumière et au photodétecteur, de telle sorte que le photodétecteur détecte un rayonnement réfléchi ou rétrodiffusé par l'échantillon, l'image acquise par le photodétecteur étant alors une image dite de réflexion. Cela s'adresse plus particulièrement aux échantillons épais, pour lesquelles une configuration en transmission conduirait à une atténuation trop importante du rayonnement lumineux transmis par l'échantillon.

Le photodétecteur peut être situé à une distance de l'échantillon inférieure à 1 cm, ce qui permet d'obtenir un dispositif compact.

Selon un mode de réalisation, il n'y a aucune optique de grossissement entre l'échantillon et le photodétecteur. Le dispositif est alors de conception simple et peu onéreuse.

## FIGURES

La figure 1 représente un dispositif pour estimer la quantité d'un analyte dans un échantillon liquide.

La figure 2 représente trois mesures du spectre d'absorption d'un indicateur coloré, en l'occurrence le Formazan, utilisé dans un mode de réalisation.

Les figures 3A et 3B représentent des images obtenues lors d'un exemple de réalisation, sur un échantillon comprenant du sang total, pour deux concentrations différentes de glucose dans l'échantillon.

Les figures 4A et 4B représentent des résultats comparatifs de mesures.

La figure 5 représente les étapes d'un mode de réalisation d'un procédé selon l'invention.

La figure 6 représente les étapes d'une variante de ce mode de réalisation.

Les figures 7A, 7B, 7C, 7D et 7E représentent des images de l'échantillon à différents instants, respectivement 10 s, 20 s, 30 s, 60 s, 120 s suivant l'introduction de l'échantillon dans une chambre microfluidique, l'échantillon comportant une concentration de glucose égale à 4.8 mM.

La figure 7F représente l'image d'un échantillon, 120 s après son introduction dans une chambre microfluidique, l'échantillon comportant une concentration de glucose égale à 19 mM.

Les figures 8A, 8B et 8C représentent, à partir d'images obtenues à l'aide d'un échantillon présentant une concentration de glucose de 4.8 mM, l'évolution d'un indicateur, dit indicateur de lyse, en fonction du temps. Cet indicateur de lyse est respectivement l'écart-type de l'intensité dans chaque image, la moyenne de l'intensité d'une image de comparaison, formée par la différence entre deux images successives, et l'écart-type de l'intensité dans ladite image de comparaison.

Les figures 9A, 9B et 9C représentent des figures respectivement analogues aux figures 8A, 8B et 8C pour un échantillon présentant une concentration de glucose de 19 mM.

## EXPOSE DE MODES DE REALISATION PARTICULIERS

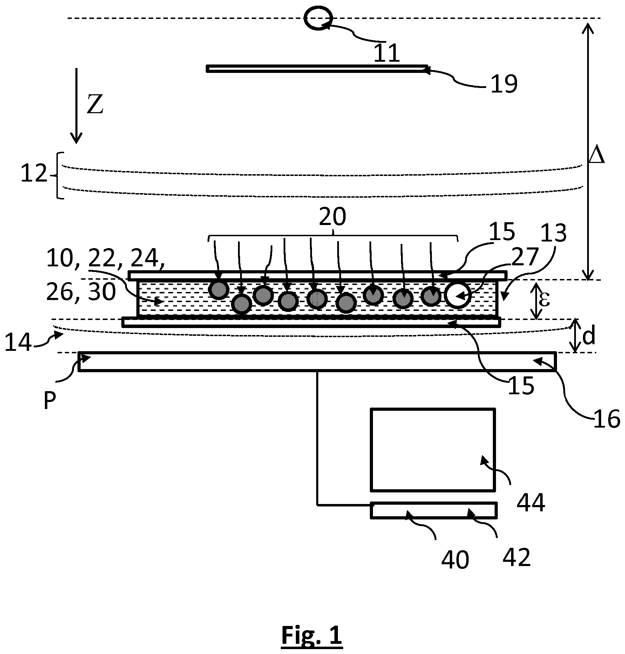

La figure 1 représente un exemple d'un dispositif pour estimer la quantité d'un analyte dans un échantillon liquide. Une source de lumière 11 est apte à produire un rayonnement lumineux 12, ou rayonnement lumineux incident, dans une bande spectrale d'illumination, en direction d'un échantillon 10, selon un axe de propagation Z. L'échantillon 10 comporte un liquide ainsi que des particules 20 baignant dans ce liquide. Le liquide peut notamment être un liquide corporel, par exemple du sang. Il peut notamment s'agir de sang total. Les particules 20 peuvent être des particules sanguines, et plus particulièrement des globules rouges.

La distance $\Delta$ entre la source de lumière et l'échantillon est de préférence supérieure à 1 cm. Elle est de préférence comprise entre 1 et 30 cm, typiquement 5 cm.

La source de lumière 11 peut être une diode électroluminescente ou une source de lumière laser, par exemple une diode laser.

De préférence, la source de lumière, vue par l'échantillon, est considérée comme ponctuelle, mais cela n'est pas nécessaire. Le terme ponctuel désigne le fait que son diamètre (ou sa diagonale) doit être inférieure au cinquième, mieux au dixième de la distance entre l'échantillon et la source de lumière. Ainsi, le rayonnement lumineux 12 parvient à l'échantillon sous la forme d'ondes planes, ou pouvant être considérées comme telles.

La source de lumière 11 peut être associée à un diaphragme 18, non représenté sur la figure 1 de façon à apparaître ponctuelle. L'ouverture du diaphragme est typiquement comprise entre 50 $\mu$m et 1mm, de préférence 50 $\mu$m et 500 $\mu$m. La présence d'un diaphragme n'est pas nécessaire.

La source de lumière 11 peut également être fibrée. Dans ce cas, une fibre optique s'étend entre une première extrémité, disposée face à une source lumineuse, et collectant la lumière de cette dernière, et une deuxième extrémité, émettant la lumière vers l'échantillon. Dans ce cas, cette deuxième extrémité est considérée comme étant la source de lumière 11.

La source de lumière 11 peut comporter un filtre optique 19, notamment un filtre passe-bande, permettant d'ajuster la bande spectrale d'illumination du rayonnement lumineux 12 de la source de lumière 11. La bande spectrale d'illumination du rayonnement lumineux émis par la source de lumière 11 est adapté à un spectre d'absorption d'un indicateur coloré 24 décrit dans la suite de la description.

L'échantillon 10 est contenu dans une chambre fluidique 13. Les parois latérales de la chambre ne sont pas représentées. La chambre fluidique 13 est par exemple une microcuvette, d'utilisation courante dans les dispositifs de type point of care, dans laquelle l'échantillon 10 pénètre par capillarité. Dans la figure 1, on a représenté deux parois longitudinales 15, transparentes et distantes de 150 $\mu$m. La distance entre ces deux parois longitudinales 15, selon l'axe de propagation Z, correspond à l'épaisseur $\varepsilon$ de l'échantillon. Cette dernière varie typiquement entre 20 $\mu$m et 1 cm, et est de préférence comprise entre 50 $\mu$m et 500 $\mu$m, par exemple 150 $\mu$m.

L'échantillon 10 est disposé entre la source de lumière 11 et un photodétecteur matriciel 16, ou capteur d'image, apte à établir une image I, dite image de transmission, d'un faisceau lumineux 14 transmis par l'échantillon 10. Le photodétecteur matriciel s'étend selon un plan de détection P, de préférence parallèlement, ou sensiblement parallèlement aux parois longitudinales 15 de la chambre fluidique 13. Le terme sensiblement parallèlement signifie que les deux éléments peuvent ne pas être rigoureusement parallèles, une tolérance angulaire de quelques degrés, inférieure à 10° ou à 20° étant admise.

Le photodétecteur matriciel 16 comporte une matrice de pixels, de type CCD (de l'anglais Charge Coupled Device) ou un CMOS (de l'anglais Complementary Metal-Oxyde Semiconductor). Les photodétecteurs dont le pas inter pixel est inférieur à 3 μm sont préférés, car ils permettent d'obtenir des images avec une résolution spatiale satisfaisante.

De préférence, le photodétecteur comprend une matrice de pixels, au-dessus de laquelle est disposée une fenêtre de protection transparente. La distance entre la matrice de pixels et la fenêtre de protection est généralement comprise entre quelques dizaines de μm à 150 à 200 μm. De préférence, le plan de détection P selon lequel s'étend le photodétecteur est perpendiculaire à l'axe de propagation Z de l'onde lumineuse incidente 12.

On remarque, dans cet exemple, l'absence d'optique de grossissement entre le photodétecteur matriciel 16 et l'échantillon 10. Cela n'empêche pas la présence éventuelle de microlentilles de focalisation au niveau de chaque pixel du photodétecteur 16. Cela permet de former une image de transmission du faisceau 14 transmis par l'échantillon en minimisant la distance entre l'échantillon et le photodétecteur. Cela permet d'utiliser un dispositif d'analyse particulièrement simple et compact. Ainsi, en l'absence d'optique de grossissement, la distance d entre l'échantillon et les pixels du photodétecteur est de préférence inférieure à 2 cm, voire à 1 cm, et préférentiellement comprise entre 50 μm et 2 cm, de préférence comprise entre 100 μm et 2 mm.

Un processeur 40, par exemple un microprocesseur, est apte à traiter les images I acquises par le photodétecteur matriciel 16. En particulier, le processeur est un microprocesseur relié à une mémoire programmable 42 dans laquelle est stockée une séquence d'instructions pour effectuer les opérations de traitement d'image et de calcul décrites dans cette description. Cette mémoire programmable 42 peut également comporter des informations de calibration du dispositif, comme cela sera évoqué ultérieurement. Le microprocesseur peut être relié à un écran 44.

L'échantillon 10 comporte un analyte 30 dont on souhaite déterminer une quantité ou une concentration dans l'échantillon. Dans cet exemple, l'analyte est le glucose. Les principes de détection du glucose dans un échantillon sanguin par la mise en œuvre de réactions enzymatiques aboutissant à la formation d'un indicateur coloré sont décrits dans les brevets US3964974 et US5866349. D'une façon générale, cette méthode colorimétrique est basée sur :

- l'oxydation du glucose par NAD (acronyme de nicotinamide adenine dinucleotide), en présence de GDH (acronyme de glucose dehydrogenase), aboutissant à la formation de NADH + H+ (acronyme de acide dihydronicotinique amide adenine dinucleotide).

- la réduction d'un sel de tétrazolium par NADH + H+, en présence de diaphorase (dihydrolipoyl dehydrogenase), réaction aboutissant à la formation d'un indicateur coloré 24, dont la concentration est représentative de la concentration de glucose dans l'échantillon.

Le sel de tétrazolium utilisé peut être du MTT, acronyme de bromure de 3-(4,5-diméthylthiazolyl-2-yl)-2,5-diphényltetrazolium, auquel cas l'indicateur coloré est le Formazan, de couleur violette.

Le terme indicateur coloré désigne une espèce chimique ayant une couleur particulière, et dont la formation dans l'échantillon est apte à modifier le spectre d'absorption ou le spectre de transmission de l'échantillon.

Aussi, le procédé comporte une étape de mélange de l'échantillon 30 avec un premier réactif 22, permettant la formation d'un indicateur coloré 24 en réagissant avec l'analyte 30 présent dans l'échantillon, en l'occurrence le glucose. Le premier réactif 22 peut comporter de la GDH, du NAD, de la Diaphorase et du MTT.

Dans un échantillon comportant du sang total, le glucose est présent dans le plasma ainsi que dans les globules rouges 20, en suspension dans le plasma. Afin de tenir compte de la quantité de glucose présente dans les globules rouges, le procédé peut comprendre une étape d'hémolyse, en ajoutant un deuxième réactif 26, dit réactif de lyse, apte à lyser les globules rouges. Par exemple, ce deuxième réactif 26 est de la Saponine.

Mais la lyse des globules rouges 20 peut entraîner la formation de bulles d'air 27 dans l'échantillon. De ce fait, après le mélange avec le premier réactif et le deuxième réactif, l'échantillon se colore progressivement du fait de la formation de l'indicateur coloré 24. Cependant, cette coloration n'est pas spatialement homogène, en particulier du fait de la présence de bulles d'air. De ce fait, la transmission optique T de l'échantillon n'est pas homogène.

Par transmission optique, on entend une comparaison de l'intensité d'un faisceau lumineux incident à l'échantillon avec l'intensité d'un faisceau lumineux transmis par l'échantillon. La comparaison peut notamment prendre la forme d'un ratio, auquel cas

$$T^\lambda = \frac{I^\lambda}{I_0^\lambda}$$

, où :

- $T^\lambda$ désigne la transmission optique à la longueur d'onde $\lambda$ ;
- $I_0^\lambda$ désigne l'intensité du faisceau lumineux 12 incident à l'échantillon à la longueur d'onde $\lambda$ ;
- $I^\lambda$ désigne l'intensité du faisceau lumineux 14 transmis par l'échantillon à la longueur d'onde $\lambda$ ;

La formation de l'indicateur coloré 24 représentatif de l'analyte 30 entraîne une diminution de la transmission optique dans une bande spectrale d'absorption (ou bande spectrale de coloration) de l'indicateur.

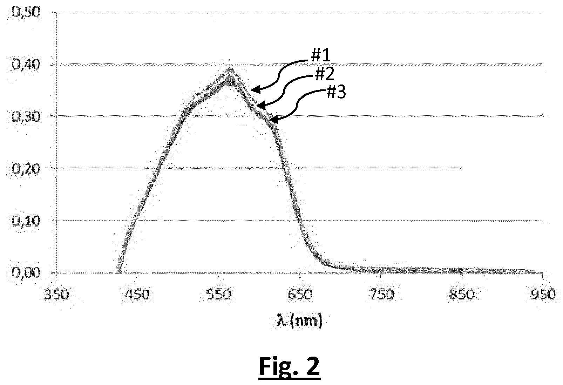

La figure 2 représente le spectre d'absorption du Formazan. Elle a été obtenue expérimentalement en faisant réagir du MTT et du NADH en présence de diaphorase dans un tampon PBS (acronyme de Phosphate Buffered Saline signifiant tampon phosphate salin) à pH 7.4 à 30°C. Le volume total réactionnel est égal à 300 microlitres et la réaction est réalisée dans une microplaque de 96 puits. Après 15 minutes de réaction, une mesure de l'absorbance DO est réalisée en utilisant un lecteur de microplaques commercialisé par Tecan afin de déterminer le spectre de l'absorbance du Formazan produit. Ainsi, lorsque l'indicateur coloré 24 mis en œuvre dans le procédé est le Formazan, la bande spectrale d'absorption s'étend entre 370 et 670 nm, avec un maximum d'absorption vers $\lambda=565$ nm. La figure 2 représente trois spectres réalisés, référencés #1, #2 et #3, ces trois spectres étant superposés les uns aux autres.

Un dispositif similaire à la figure 1 a été testé en utilisant :

- une diode laser de fabricant Thorlabs, émettant dans une longueur d'onde de 650 nm, faisant office de source d'excitation 11,
- un capteur CMOS monochrome 12bits, du fabricant Mightex sous la référence BTN-B050-U, faisant office de photodétecteur matriciel 16,
- une microcuvette fournie par Hemocue, sous la référence HE114701, agissant en tant que chambre microfluidique 13, contenant les divers réactifs nécessaires au dosage du glucose ainsi que l'échantillon.

La microcuvette a été placée entre la diode laser et le capteur CMOS, à une distance de $\Delta=5$ cm de la source laser et à une distance de d = 1 mm du capteur CMOS. L'échantillon est du sang total humain veineux prélevé sur EDTA (Ethylène Diamine Tétra-Acétique), ce dernier agissant en tant qu'agent anticoagulant.

Différents essais ont été réalisés en mélangeant le sang total à une quantité calibrée de glucose, en utilisant une solution de glucose de fournisseur Sigma Aldrich et de référence G8644.

Le sang est utilisé pour remplir la microcuvette précitée, agissant en tant que chambre microfluidique 13, par capillarité. Cette microcuvette contient, sous forme embarquée, d'une part un premier réactif 22, permettant la formation de Formazan en fonction de la concentration en glucose dans l'échantillon, ce premier réactif 22 comportant de la GDH, du NAD, du MTT et de la diaphorase. La microcuvette contient, d'autre part, un deuxième réactif 26, en l'occurrence de la saponine, pour lyser les globules rouges.

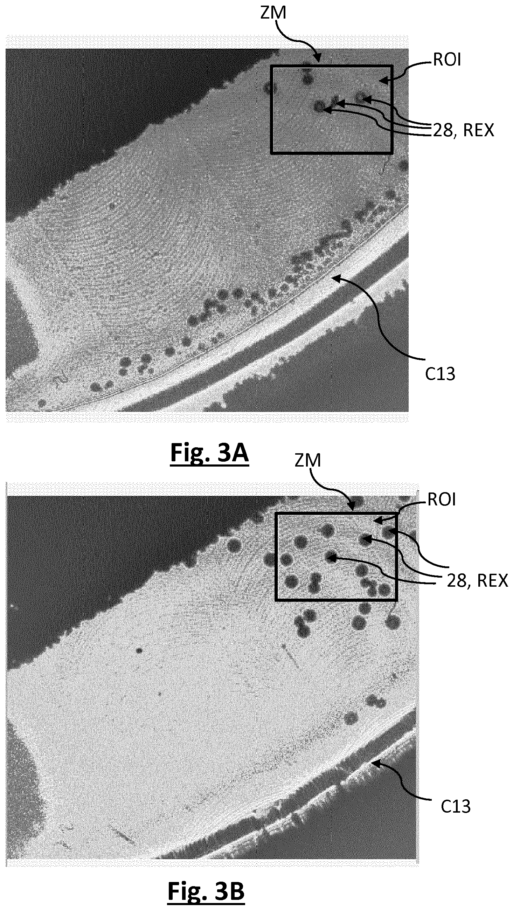

Les figures 3A et 3B représentent des images obtenues par le capteur CMOS alors que la concentration en glucose dans l'échantillon est respectivement égale à 9,4 et 2,3 mM. Chaque image est obtenue 120 secondes après l'introduction de sang dans la microcuvette, le temps de pose étant de 0.3 ms

Sur chaque figure, on peut observer les contours de la microcuvette 13, dans laquelle s'étend l'échantillon. On observe également des arcs de cercle générés par le procédé de fabrication de la microcuvette.

La quantification du glucose est réalisée sur une zone de mesure ZM de chaque image de transmission I, englobant 640 x 460 pixels. Cette zone de mesure ZM est matérialisée par un rectangle sur les figures 3A et 3B. L'intensité de la lumière collectée par le photodétecteur dépend de la transmission optique de l'échantillon analysé. Plus la concentration en glucose dans l'échantillon est importante, plus la concentration de Formazan formée dans l'échantillon est importante, plus la transmission optique de l'échantillon est faible, réduisant alors l'intensité de la lumière détectée par le photodétecteur 16.

Ainsi, plus la concentration en glucose 30 est élevée, plus l'image formée par le photodétecteur est sombre. Cette évolution de la transmission optique peut être quantifiée en utilisant une grandeur k représentative de l'intensité des pixels dans la zone de mesure ZM. Cette grandeur k peut être établie à partir de l'intégrale ou de la moyenne des pixels dans cette zone de mesure. Un étalonnage réalisé sur des solutions de calibration, dans lesquelles la concentration en glucose est connue, permet de relier la grandeur k mesurée à la concentration en glucose dans l'échantillon. Les données obtenues lors de cet étalonnage sont stockées dans la mémoire 42.

Cependant, la lyse des globules rouges s'accompagne de la formation de bulles d'air 27 dans l'échantillon, ces dernières apparaissant sous la forme de traces sombres 28, en forme de disque, sur chaque image. Leur quantité et leur position, dans la zone de mesure ZM sont aléatoires, comme le montrent les figures 3A et 3B, et évoluent avec le temps. Aussi, la transmission optique dans l'échantillon n'est pas spatialement homogène. Cette inhomogénéité peut donner lieu à des erreurs de mesure significatives.

Aussi, il est proposé d'établir une grandeur représentant l'intensité des pixels dans la zone de mesure ZM, tout en s'affranchissant de l'impact des bulles d'air. Pour cela, préalablement au calcul de la grandeur k, on définit, à l'intérieur de la zone de mesure, une région d'intérêt ROI comprenant les pixels situés dans la zone de mesure, en excluant les pixels correspondant aux traces 28 formées par les bulles d'air 27. Autrement dit, on détermine, à l'intérieur de la zone de mesure ZM, une région d'intérêt ROI et au moins une région d'exclusion REX, cette dernière étant considérée comme représentative d'une trace 28. Une zone de mesure peut comporter plusieurs régions d'exclusion, chacune correspondant à une trace 28. La région d'intérêt correspond donc à une région, dans la zone de mesure, complémentaire de chaque région d'exclusion. Le plus souvent, la zone de mesure ZM comporte une pluralité de régions d'exclusion REX, ces régions étant distinctes l'une de l'autre et isolées les unes des autres, de telle sorte que la région d'intérêt s'étend entre ces différentes régions d'exclusion. Chaque région d'exclusion est notamment délimitée par un contour fermé, notamment de forme annulaire, circulaire ou polygonale.

La grandeur est alors calculée en fonction des pixels de la région d'intérêt ainsi définie, sans prise en compte des pixels de la région d'exclusion REX ou des régions d'exclusion REX présentes dans la zone de mesure ZM.

En lien avec les figures 3A et 3B, sur chaque zone de mesure ZM, il est possible de délimiter une région d'intérêt ROI correspondant à ladite zone de mesure, à l'exclusion des régions d'exclusions REX, chaque région d'exclusion correspondant à un disque sombre. On remarque que l'intensité moyenne des pixels de la région d'intérêt associée à la figure 3A est significativement plus faible que l'intensité moyenne des pixels de la région d'intérêt associée à la figure 3B, cela en raison de la plus forte concentration en glucose sur l'échantillon représenté sur la figure 3A, se traduisant par une transmission optique plus faible.

Des essais comparatifs ont été réalisés, sur des échantillons A, B, C, D et E dont la concentration en glucose est respectivement égale à 0,4 mM ; 2,3 mM ; 4,8 mM ; 9,4 mM et 19 mM.

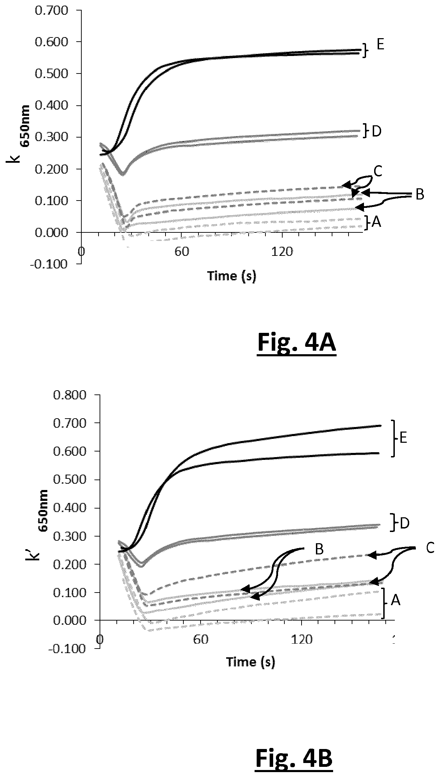

Sur chaque échantillon, on a reproduit le protocole décrit en lien avec les figures 3A et 3B. Une grandeur représentant l'intensité du rayonnement transmis par l'échantillon est déterminée à partir d'un instant initial où l'échantillon pénètre dans la chambre fluidique, qui correspond à l'instant initial $t = t_0 = 0$. La grandeur est calculée durant une période de 170 s après l'instant initial. Les figures 4A et 4B représentent l'évolution de la grandeur en fonction du temps, sachant que :

- la grandeur k, dont l'évolution temporelle est représentée sur la figure 4A, correspond à l'intensité moyenne sur région d'intérêt ROI correspondant à la zone de mesure ZM, après exclusion de chaque région d'exclusion REX, représentative des traces 28. Cette intensité moyenne est normalisée par l'intensité moyenne, sur la zone de mesure ZM, d'une image de référence $I_0$ obtenue sans échantillon entre la source de lumière et le photodétecteur. Du fait de cette normalisation, la grandeur k correspond à la moyenne de la transmission optique, telle que précédemment définie, dans la région d'intérêt ROI.

- La grandeur k', dont l'évolution temporelle est représentée sur la figure 4B, correspond à l'intensité moyenne sur la zone de mesure ZM. Cette intensité moyenne est normalisée par l'intensité moyenne, sur la zone de mesure ZM, d'une image de référence $I_0$ obtenue sans échantillon entre la source de lumière et le photodétecteur. Du fait de cette normalisation, la grandeur k' correspond à la moyenne de la transmission optique, telle que précédemment définie, dans la région zone de mesure ZM.

Dans cet exemple, la région d'intérêt est obtenue en appliquant un seuillage en intensité des pixels de la zone de mesure. Les pixels dont l'intensité dépasse un certain seuil sont considérés comme appartenant à la région d'intérêt ROI, les autres étant considérés comme appartenant à une région d'exclusion REX.

Autrement dit, la grandeur représentée sur la figure 4A peut être exprimé sous la forme :

$$ k = \frac{\dfrac{\sum_{r \in ROI} I(r)}{N_{r \in ROI}}}{\dfrac{\sum_{r \in ZM} I_0(r)}{N_{r \in ZM}}} $$

où :

- r désigne la position d'un pixel dans l'image I ;
- I(r) désigne l'intensité du pixel r de l'image I ;
- $I_0(r)$ désignant l'intensité du pixel r de l'image de référence $I_0$;
- $N_{r \in ROI}$ désigne le nombre de pixels contenus dans la région d'intérêt ROI.

La grandeur représentée sur la figure 4B peut être exprimé sous la forme :

$$ k' = \frac{\dfrac{\sum_{r \in ZM} I(r)}{N_{r \in ZM \geq P}}}{\dfrac{\sum_{r \in ZM} I_0(r)}{N_{r \in ZM}}} $$

- $N_{r \in ZM}$ désignant le nombre de pixels contenus dans la zone de mesure ZM.

La comparaison des figures 4A et 4B montre que la grandeur k est préférable à la grandeur k'. En effet, la grandeur k', dont l'évolution temporelle est représentée sur la figure 4B, évolue constamment avec le temps, et peut prendre des valeurs sensiblement différentes pour une même quantité d'analyte. C'est notamment le cas des échantillons A, B et E présentant respectivement une concentration en glucose de 0.4 mM, 4.8 mM et 19 mM. A l'inverse, la grandeur k, dont l'évolution temporelle est représentée sur la figure 4A, apparaît plus fiable : son évolution en fonction du temps est moins marquée, au-delà d'une durée de 60 s après l'instant initial. De plus, les mesures correspondant à une même concentration de glucose sont plus répétitives.

On comprend que le fait d'acquérir une image, et non pas un signal optique monodimensionnel, non spatialement résolu, comme dans l'art antérieur, permet d'identifier les traces 28, et de les exclure lors de l'estimation de la concentration de glucose. Cela permet d'obtenir une estimation plus fiable de la quantité de glucose dans le sang, du fait de la non prise en compte, dans la partie de l'image analysée, des traces 28, non représentatives de la coloration observée. Par ailleurs, au-delà des traces liées aux bulles d'air produites par l'hémolyse, le fait de disposer d'une image I permet d'exclure toute autre perturbation, par exemple un défaut de fabrication sur une chambre fluidique 13, ou encore une poussière à l'intérieur ou à l'extérieur de la cette chambre.

On constate également que le recours à une deuxième source de lumière, illuminant l'échantillon dans une longueur d'onde de 880nm, n'est pas nécessaire.

Ainsi, sur la base d'une image acquise par le photodétecteur, et à l'aide d'une seule source de lumière, il est possible d'estimer la teneur en glucose d'un échantillon sanguin, et, de façon plus générale, de doser un analyte présent dans un échantillon liquide, par l'observation d'une coloration de l'échantillon résultant de la transformation de cet analyte.

Dans cet exemple, la détermination de la région d'intérêt ROI est réalisée par seuillage d'image, les pixels appartenant à la région d'intérêt lorsque leur intensité est supérieure à un seuil. La valeur de ce seuil a été prise égale à 50 pour les échantillons A, B, C, 30 pour l'échantillon D et 20 pour l'échantillon E.

Ce seuil peut être prédéterminé ; il peut également être défini, au cas par cas, sur la base de chaque image, par exemple par une analyse d'histogramme dans la zone de mesure ZM. En effet, les pixels correspondant à une région d'exclusion forment un pic dans l'histogramme ; le seuil peut être déterminé en se basant sur une intensité déterminée à partir de ce pic, par exemple une borne de ce pic, notamment la borne supérieure lorsqu'une région d'exclusion apparaît sous la forme d'une trace sombre.

D'autres méthodes pour déterminer la région d'intérêt ROI sont envisageables, basées sur des techniques connues d'analyse morphologique et de segmentation d'image. On tire alors profit du fait que chaque région d'exclusion REX correspond à une forme fermée, sensiblement circulaire. Des algorithmes de reconnaissance de forme ou de contours peuvent donc être mis en œuvre pour identifier et délimiter chaque région d'exclusion.

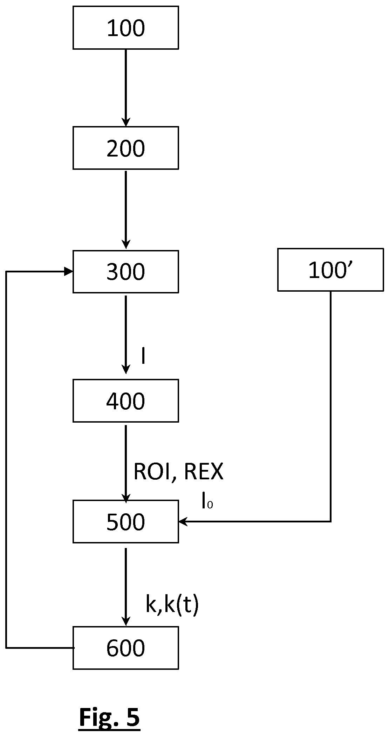

La figure 5 résume les principales étapes d'un procédé d'estimation d'une concentration de glucose selon l'invention.

Au cours d'une étape 100, le liquide corporel est introduit dans une chambre fluidique 13. Le liquide introduit dans la chambre forme l'échantillon à analyser 10.

Au cours d'une étape 200, le liquide est mélangé à un premier réactif 22 apte à former un indicateur coloré 24 en présence de glucose 30, et à un deuxième réactif 26 apte à lyser les globules rouges présents dans l'échantillon. Le premier et le deuxième réactifs peuvent notamment être présents dans ladite chambre fluidique à l'état sec, par exemple sous forme lyophilisée.

Au cours d'une étape 300, la chambre fluidique est illuminée par une source de lumière 11 et le rayonnement lumineux 14 transmis par l'échantillon est collecté par un photodétecteur matriciel de façon à en former une image de transmission I. Cette image de transmission peut être formée après une période temporelle T prédéterminée suivant l'introduction de l'échantillon dans la chambre fluidique.

Au cours d'une étape 400, l'image de transmission I est analysée, de façon à déterminer, dans une zone de mesure ZM de ladite image, une région d'intérêt ROI et une ou plusieurs région(s) d'exclusion (REX).

Au cours d'une étape 500, une grandeur k, représentative de l'intensité des pixels dans la région d'intérêt à l'instant T est calculée à partir de l'image de transmission I, les pixels de chaque région d'exclusion n'étant pas pris en compte dans le calcul de la grandeur k.

Au cours d'une étape 600, la grandeur k est comparée à des grandeurs étalons, obtenues sur des solutions étalons, dans des conditions expérimentales analogues, de façon à estimer la quantité ou la concentration de l'analyte 30. Les grandeurs étalons sont stockées dans une mémoire 42 est reliée au processeur 40.

L'étape 500 peut nécessiter l'acquisition d'une image $I_0$, dite image initiale, formée par le photodétecteur en l'absence de liquide dans la chambre fluidique, ou en l'absence de chambre fluidique. Cette image de référence $I_0$ peut être établie au cours d'une étape 100', sans chambre fluidique entre la source de lumière 11 et le photodétecteur 16, avant ou après la formation de l'image I. Elle peut également être établie en disposant une chambre fluidique sans échantillon entre la source de lumière 11 et le photodétecteur 16.

Les étapes 100 et 200 peuvent être interverties ou réalisées simultanément.

De façon alternative, les étapes 300 à 600 sont répétées à différents instants de telle sorte qu'au cours de l'étape 600, on détermine une pluralité de grandeurs k en fonction du temps t, la quantité ou la concentration d'analyte étant estimée à partir de l'évolution temporelle k(t) de ladite grandeur.

Selon un autre mode de réalisation, la source de lumière 11 et le photodétecteur 16 sont disposés du même côté par rapport à l'échantillon 10. Le photodétecteur détecte alors une image I' du rayonnement 14' ré-

fléchi, et éventuellement rétrodiffusé par l'échantillon. La formation d'un indicateur coloré 24 modifie les propriétés d'absorption optique $A_\lambda$ de l'échantillon, notamment dans la bande spectrale d'absorption de l'indicateur coloré 24. Précisons que l'absorption optique, à une longueur d'onde $\lambda$ donnée, peut être définie par l'expression : $A^\lambda = 1 - T^\lambda$, $T^\lambda$ étant la transmission optique précédemment définie.

L'avantage de ce mode de réalisation est qu'il peut être appliqué à des échantillons plus épais, par exemple dont l'épaisseur dépasse 5 mm voire 1 cm.

Selon une variante du mode de réalisation préalablement décrit, préalablement à la mise en œuvre de l'étape 400, on cherche à s'assurer que la lyse des globules rouges est suffisamment avancée pour que l'image analysée lors des étapes suivantes ne soit pas influencée par la diffusion du rayonnement lumineux 12 par ces globules rouges. Autrement dit, on attend un instant $T_l$, dit instant de lyse, au-delà duquel la lyse est considérée comme étant réalisée.

En effet, tant que la lyse n'a pas atteint un stade suffisamment avancé, la mesure du rayonnement transmis 14 est influencée du fait de la diffusion par les globules rouges dans l'échantillon. Cela peut causer une erreur de mesure. Pour s'en prémunir, il est préférable d'attendre que la lyse soit achevée, ou suffisamment avancée pour que la transmission de la lumière à travers l'échantillon soit représentative de l'absorption optique de l'échantillon, l'influence de la diffusion étant alors négligeable du fait de la faible quantité résiduelle de globules rouges.

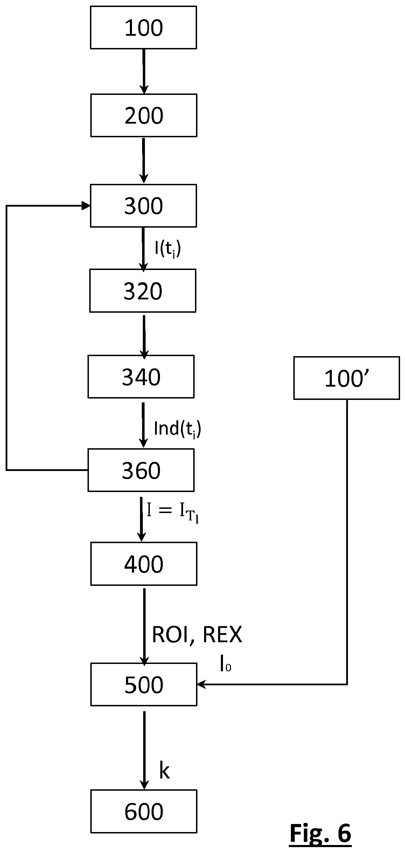

Cette variante est représentée sur la figure 6. Au cours d'une étape 300, la chambre fluidique est illuminée par la source de lumière 11 et le rayonnement lumineux 14 transmis par l'échantillon est collecté par un photodétecteur matriciel de façon à former une image de transmission $I(t_i)$ acquise à l'instant $t_i$. La variable $t_i$ est une variable temporelle, représentant un temps échantillonné.

Au cours d'une étape 320, on détermine, dans l'image $I(t_i)$, une zone de contrôle de lyse ZC. Cette zone de contrôle correspond à tout ou partie de l'échantillon liquide analysé, dans la chambre fluidique.

Au cours d'une étape 340, on établit un indicateur de lyse $Ind(t_i)$ déterminé à l'aide de l'intensité des pixels de la zone de contrôle de lyse. Cet indicateur de lyse est par exemple la variance ou l'écart-type de la distribution de l'intensité de ces pixels.

Au cours d'une étape 360, on détermine un niveau de lyse à partir de la valeur de l'indicateur $Ind(t_i)$. En fonction de sa valeur, on estime que la lyse est suffisamment avancée ou on réitère les étapes 300 à 360, en considérant une image $I(t_{i+1})$, acquise à un instant $t_{i+1}$ postérieur à l'instant $t_i$. Lorsque la valeur de l'indicateur de lyse atteint un certain critère, dit critère de lyse, on considère qu'on a atteint un instant de fin de lyse $T_l$ et on passe aux étapes suivantes 400 à 600, soit en acquérant une autre image, soit en se basant sur l'image

$I(t=T_l)$ acquise à l'instant $T_l$.

L'indicateur de lyse peut être établi image par image, à chaque image $I(t_i)$ étant associé un indicateur de lyse $Ind(t_i)$.

Il peut également être représentatif d'une corrélation ou d'une différence entre deux images successives $I(t_i)$ et $I(t_{i-1})$, un indicateur $Ind(t_i)$ attribué au couple d'image $I(t_i)$ , $I(t_{i-1})$. Dans ce cas, au cours de l'étape 340, on détermine par exemple une différence d'image $\Delta(t_i)$ telle que $\Delta(t_i) = I(t_i) - I(t_{i-1})$. Cette image $\Delta(t_i)$ est appelée image de comparaison à l'instant $t_i$. L'indicateur de lyse $Ind(t_i)$ peut correspondre à la moyenne ou à l'écart type, ou encore la variance de ladite image de comparaison $\Delta(t_i)$.

Le critère de fin de lyse est déterminé en fonction de l'évolution temporelle de l'indicateur de lyse $Ind(t_i)$. En particulier, on considère que le critère de fin de lyse est atteint lorsque l'indicateur de lyse n'évolue plus significativement. Le critère de fin de lyse peut également être une valeur seuil prédéterminée, l'instant de fin de lyse $T_l$ étant considéré comme atteint lorsque l'indicateur de lyse $Ind(t_i)$ franchit un tel seuil.

Les autres étapes 100, 200, 400, 500, 600 sont analogues aux étapes décrites en lien avec la figure 5. La zone de contrôle ZC définie lors de l'étape 320 peut être similaire à zone de mesure ZM décrite en lien avec l'étape 400.

Des essais expérimentaux ont été réalisés, afin de déterminer, par analyse d'images, un instant de fin de lyse $T_l$ , et cela dans différentes conditions.

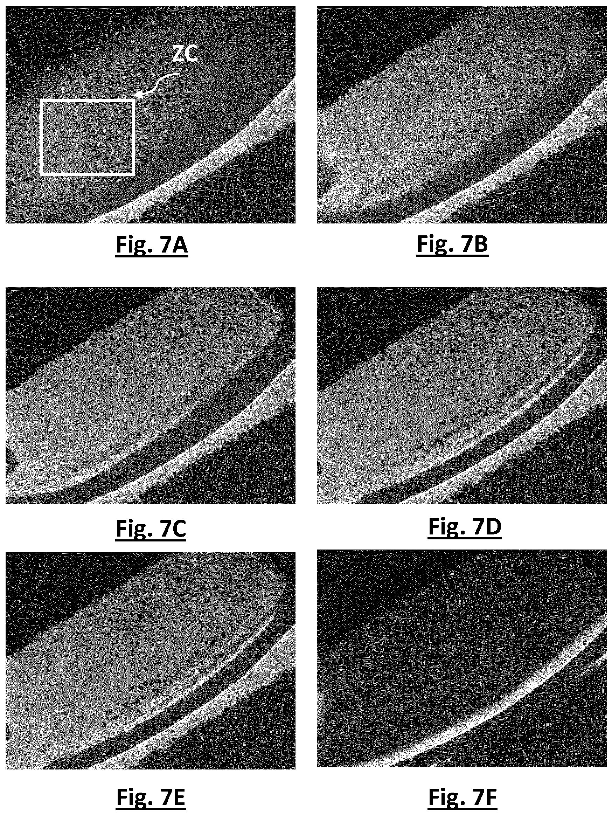

Les images des figures 7A, 7B, 7C, 7D et 7E représentent un échantillon similaire à celui décrit dans les figures 3A et 3B, la concentration en glucose étant de 4.8 mM. On a sélectionné une zone de contrôle ZC, de 640 x 480 pixels. Cette zone est représentée par un cadre blanc sur l'image 7A, sachant que cette zone est identique pour chacune des images 7A à 7E. Si $t_0$ désigne l'introduction de l'échantillon dans la microcuvette, ces images ont été respectivement acquises à $t_0 + 10$ s, $t_0 + 20$ s, $t_0 + 30$ s, $t_0 + 60$ s, $t_0 + 120$ s.

On constate qu'à $t_0 + 10$ s (figure 7A), l'image apparaît bruitée, notamment en comparaison avec les autres images, ce bruit étant dû à la diffusion du rayonnement lumineux incident par les globules rouges. On comprend que le signal mesuré par le photodétecteur est grandement influencé par la diffusion. Au fur et à mesure que le temps s'écoulant depuis l'introduction augmente, les images sont de moins en moins bruitées, et ceci est particulièrement net entre $t_0 + 10$ s (figure 7A) et $t_0 + 30$ s (figure 7C). Au-delà de $t_0 + 30$ s, on observe la formation de bulles, apparaissant sous la forme de disques noirs, dont la surface augmente au cours du temps. Cette augmentation de la surface peut notamment s'apprécier en comparant les figures 7C à 7E. Les inventeurs considèrent que la lyse peut être considérée comme achevée, ou n'évoluant plus significativement, après $t_0 + 30$ s.

La figure 7F représente un échantillon dont la concentration en glucose s'élève à 19 mM, l'image étant acquise à $t_0$ + 120 s. On observe que le niveau de gris est bien plus sombre que sur l'image 7E, réalisée dans les mêmes conditions, avec une teneur de glucose plus faible. Cela est dû au fait que l'intensité du faisceau lumineux transmis est très majoritairement gouvernée par l'absorption due à l'indicateur coloré, la concentration de ce dernier étant plus importante sur la figure 7F que sur la figure 7E du fait de la différence de quantité de glucose.

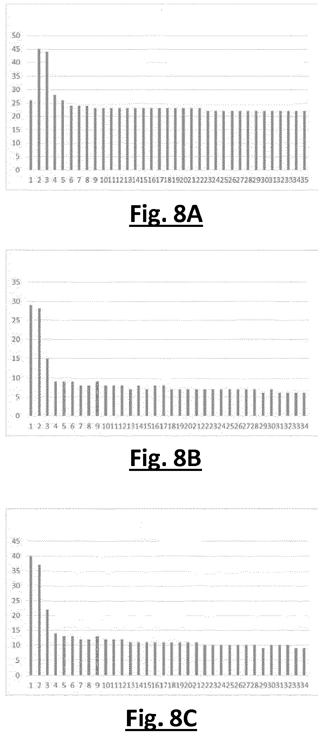

Sur la figure 8A, on a représenté l'évolution de l'écart-type de l'intensité des pixels dans la zone de contrôle de lyse ZC en fonction du temps. Plus précisément, l'axe des abscisses représente le numéro d'incrémentation de chaque image analysée, l'intervalle temporel entre chaque image étant de 5 secondes. L'image 1 correspond à $t_0$ + 10s, l'image 2 correspond à $t_0$ + 15s etc...La zone de contrôle de lyse ZC est la même pour chaque image, et correspond à celle représentée sur la figure 7A.

L'écart-type fluctue dans les premières images, puis se stabilise après l'image i = 5, correspondant à une durée de 30 secondes après l'introduction de l'échantillon dans la microcuvette 30. A partir de cet instant, on considère que la lyse est achevée, ou du moins suffisamment avancée pour que les mesures ne soient pas significativement perturbées par la diffusion. Aussi, l'écart-type de chaque image peut constituer un indicateur de lyse.

La figure 8B représente l'évolution de la moyenne, dans la zone de contrôle de lyse, réalisée à partir d'images de comparaison $\Delta(t_i)$, chaque image de comparaison étant obtenue, dans cet exemple, par une soustraction d'une image $I(t_i)$ acquise à un instant $t_i$ à une image $I(t_{i-1})$ acquise à un instant $t_{i-1}$. De même que dans l'exemple décrit en lien avec la figure 8A, cette figure est réalisée à partir d'images successivement acquises selon un intervalle temporel de 5s, l'image 1 correspondant à $t_0$ + 10s. L'axe des abscisses représente le numéro i de chaque image de comparaison $\Delta(t_i)$ analysée, l'intervalle temporel entre chaque image étant de 5 secondes. L'axe des ordonnées représente la valeur moyenne de l'intensité des pixels dans la zone de contrôle de lyse ZC de chaque image de comparaison $\Delta(t_i)$. On constate une stabilisation de la valeur moyenne à partir de l'image de comparaison correspondant à l'indice i = 4, soit $t_0$ + 25s. Aussi, la moyenne de l'intensité des pixels d'images de comparaison $\Delta(t_i)$, acquises à des instants $t_i$ différents, peut constituer un indicateur de lyse.

La figure 8C représente l'évolution de l'écart type, dans la zone de contrôle de lyse ZC, réalisée à partir des images de comparaison $\Delta(t_i)$ décrites en lien avec la figure 8B. On constate une stabilisation de l'écart type à partir de l'image correspondant à l'indice i = 4, soit $t_0$ + 25s. Aussi, l'écart-type de l'intensité des pixels d'images de comparaison $\Delta(t_i)$, acquises à des instants $t_i$ différents, peut constituer un indicateur de lyse.

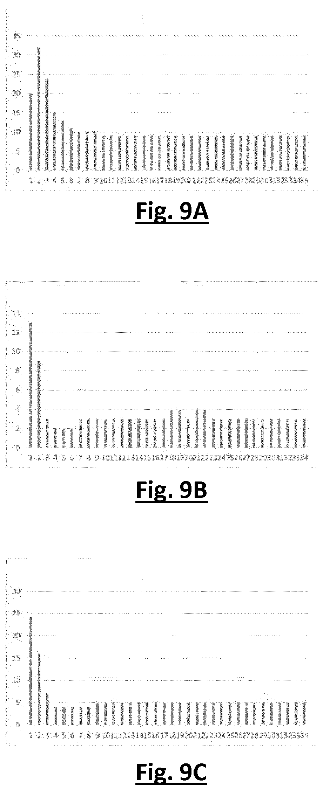

Les figures 9A, 9B et 9C représentent respectivement des résultats obtenus de la même façon que ceux représentés sur les figures 8A, 8B et 8C. Ces figures ont été obtenues en utilisant un échantillon dont la concentration en glucose s'élève à 19 mM.

De même que dans les figures 8A, 8B et 8C, et d'une façon générale, l'indicateur de lyse permet de déterminer un instant de fin de lyse, à partir duquel la lyse des particules de l'échantillon est suffisamment avancée de telle sorte que l'image acquise par le photodétecteur soit peu ou pas influencée par la diffusion, par les particules, du rayonnement lumineux 12. A partir de cet instant, l'image est majoritairement influencée par l'absorption du rayonnement lumineux 12 par l'échantillon. En l'occurrence, dans les exemples préalablement exposés, cet instant est de l'ordre de 25 à 30 secondes après l'introduction de l'échantillon dans la microcuvette.

Cet instant, dit instant de lyse, est atteint lorsque l'indicateur de lyse respecte un certain critère, dit critère de lyse. Le critère de lyse peut être une valeur seuil de l'indicateur. Il peut également s'agir une valeur représentant une stabilisation l'évolution temporelle de l'indicateur, à partir d'une comparaison entre des indicateurs établis selon plusieurs images consécutives. Le terme comparaison peut désigner une soustraction ou un ratio.

Ainsi, d'une façon générale, un objet de l'invention est un procédé de détermination d'un indicateur de lyse dans un échantillon liquide, l'échantillon comportant des particules, le procédé comportant le mélange de l'échantillon avec un réactif de lyse, apte à lyser lesdites particules, le procédé comportant les étapes suivantes :

- illumination dudit échantillon 10 à l'aide d'une source de lumière 11, apte à émettre un rayonnement lumineux 12 vers l'échantillon,
- acquisition, à l'aide d'un photodétecteur matriciel 16, d'une image I d'un rayonnement lumineux transmis ou réfléchi par l'échantillon,
- détermination d'un indicateur de lyse à partir de ladite image.

Cet indicateur de lyse représente un état d'avancement de la lyse des particules dans l'échantillon, sous l'effet du réactif de lyse. Lorsque cet indicateur de lyse atteint un certain critère, dit critère de lyse, on considère que la plupart des particules ont été lysées. L'effet de diffusion du rayonnement lumineux par l'échantillon est alors négligeable. L'image acquise par le détecteur représente alors l'absorption du rayonnement lumineux transmis ou réfléchi par l'échantillon.

L'indicateur de lyse peut comprendre :

- un terme représentant la dispersion de l'intensité des pixels d'une image acquise par le photodétecteur autour d'une valeur moyenne, par exemple une variance ou un écart-type de cette image ;
- l'établissement d'une comparaison, par exemple sous la forme d'une différence, ou d'un facteur de corrélation, entre deux images acquises à des instants différents $t_i$ et $t_{i-1}$

- un terme, représentant une valeur moyenne d'une image de comparaison $\Delta(t_i)$ résultant d'une telle comparaison, ou représentant une dispersion autour de cette valeur moyenne, par exemple une variance ou un écart-type de cette image de comparaison.

Le procédé décrit ci-dessus, appliqué à la détermination d'une concentration de glucose dans le sang total, peut être généralisé à tout analyte présent dans un échantillon liquide, en particulier un échantillon corporel.

**Revendications**

1. Procédé d'estimation d'une quantité d'un analyte (30) dans un échantillon liquide (10), comportant des particules (20), le procédé comportant les étapes suivantes :

   a) mélange dudit échantillon (10) avec un premier réactif (22) apte à former un indicateur coloré (24) en présence dudit analyte, dans l'échantillon,
   b) suite à ce mélange, illumination dudit échantillon (10) à l'aide d'une source de lumière (11), apte à émettre un rayonnement lumineux (12) vers l'échantillon,
   c) acquisition, à l'aide d'un photodétecteur matriciel (16), d'une image (I, I') d'un rayonnement lumineux (14, 14') transmis ou réfléchi par l'échantillon,
   d) estimation d'une quantité dudit analyte (30) en fonction de ladite image, selon les sous étapes suivantes :

   i) sélection d'une zone de mesure (ZM), dans ladite image, ladite zone de mesure comprenant une pluralité de pixels,

   le procédé étant **caractérisé en ce qu'**il comporte également :

   ii) détermination, à l'intérieur de la zone de mesure (ZM), d'une région d'intérêt (ROI) et d'au moins une région d'exclusion (REX),
   iii) estimation de ladite quantité d'analyte (30) à partir d'une grandeur (k) représentative de l'intensité des pixels dans ladite région d'intérêt, sans prise en compte de l'intensité des pixels de chaque région d'exclusion,

   le procédé comportant également un mélange de l'échantillon avec un deuxième réactif, dit réactif de lyse (26), apte à lyser lesdites particules, chaque région d'exclusion correspondant à la trace (28) d'une bulle d'air (27), ladite bulle d'air étant formée suite à la lyse desdites particules.

2. Procédé selon la revendication 1, dans lequel la sous-étape ii) comprend la détermination d'au moins une région d'exclusion (REX), ladite région d'intérêt (ROI) correspondant à une partie de ladite zone de mesure (ZM) ne comportant pas chaque région d'exclusion ainsi déterminée.

3. Procédé selon l'une des revendications 1 ou 2, comportant également, lors de l'étape d),

   - la détermination d'un indicateur de lyse (Ind($t_i$)) à partir de l'image acquise lors de l'étape c),
   - l'estimation de la quantité d'analyte étant réalisée lorsque cet indicateur de lyse satisfait à un critère de lyse prédéterminé.

4. Procédé selon la revendication 3, dans lequel lorsque l'indicateur de lyse ne satisfait pas audit critère de lyse, l'étape c) est réitérée, de telle sorte qu'une pluralité d'indicateurs de lyse (Ind($t_i$)) sont déterminés, à partir d'images (I($t_i$), (I($t_{i-1}$)) acquises à différents instants ($t_i$, $t_{i-1}$).

5. Procédé selon la revendication 4, dans lequel chaque indicateur de lyse est déterminé en formant une image de comparaison ($\Delta t_i$), représentant une comparaison entre deux images (I($t_i$), (I($t_{i-1}$)) acquises à différents instants ($t_i$, $t_{i-1}$).

6. Procédé selon l'une quelconque des revendications précédentes, comprenant une étape d'introduction de l'échantillon dans une chambre fluidique (13), les étapes c) et d) étant effectuées à partir d'un instant (T) prédéterminé après ladite introduction.

7. Procédé selon l'une quelconque des revendications précédentes, comprenant l'acquisition d'une pluralité d'images (I, I') successives du rayonnement lumineux transmis ou réfléchi par l'échantillon, la quantité d'analyte étant déterminée, lors de l'étape d), en fonction de l'évolution de l'intensité de chaque image en fonction du temps.

8. Procédé selon l'une quelconque des revendications précédentes, dans lequel, lors de l'étape d), ladite grandeur (k) représentative de l'intensité des pixels dans ladite région d'intérêt (ROI) comporte l'intégrale ou la moyenne des pixels dans ladite région d'intérêt.

9. Procédé selon l'une quelconque des revendications précédentes, dans lequel la région d'exclusion (REX) est déterminée par seuillage de ladite image (I, I'), ou par segmentation de ladite image (I, I').

10. Procédé selon l'une quelconque des revendications précédentes, dans lequel lesdites particules (20) sont des globules rouges, ou que l'analyte (30) est le glucose.

11. Procédé selon l'une quelconque des revendications précédentes, dans lequel le photodétecteur matriciel (16) est situé à une distance (d) de l'échantillon inférieure à 1 cm.

12. Procédé selon l'une quelconque des revendications précédentes, dans lequel il n'y a aucune optique de grossissement entre l'échantillon (10) et le photodétecteur matriciel (16).

13. Procédé selon l'une quelconque des revendications précédentes, dans lequel l'échantillon (10) est disposé entre la source de lumière (11) et le photodétecteur matriciel (16), de telle sorte que le photodétecteur matriciel détecte un rayonnement (14) transmis par l'échantillon (10), l'image acquise par le photodétecteur matriciel étant alors une image (I) dite de transmission.

14. Procédé selon l'une quelconque des revendications 1 à 12, dans lequel l'échantillon est disposé face à la source de lumière (11) et au photodétecteur matriciel (16), de telle sorte que le photodétecteur matriciel détecte un rayonnement (14') réfléchi ou rétrodiffusé par l'échantillon (10), l'image acquise par le photodétecteur matriciel étant alors une image (I') dite de réflexion.

**Patentansprüche**

1. Verfahren zur Schätzung einer Menge eines Analyten (30) in einer flüssigen Probe (10), die Partikel (20) beinhaltet, wobei das Verfahren die folgenden Schritte beinhaltet:

    a) Mischen der Probe (10) mit einem ersten Reagens (22), das geeignet ist, in Gegenwart des Analyten in der Probe einen farbigen Indikator (24) zu bilden,
    b) nach diesem Mischen Beleuchten der Probe (10) mithilfe einer Lichtquelle (11), die geeignet ist, eine Lichtstrahlung (12) zur Probe hin auszusenden,
    c) Erfassen, mithilfe eines Matrix-Fotodetektors (16), eines Bildes (I, I') einer Lichtstrahlung (14, 14'), die von der Probe durchgelassen oder reflektiert wird,
    d) Schätzen einer Menge des Analyten (30) in Abhängigkeit von dem Bild gemäß den folgenden Teilschritten:

        i) Auswählen einer Messzone (ZM) in dem Bild, wobei die Messzone eine Mehrzahl von Pixeln umfasst,

wobei das Verfahren **dadurch gekennzeichnet ist, dass** es ferner beinhaltet:

        ii) Bestimmen, innerhalb der Messzone (ZM), eines relevanten Bereichs (ROI) und mindestens eines Ausschlussbereichs (REX),
        iii) Schätzen der Analytmenge (30) anhand einer Größe (k), die für die Intensität der Pixel in dem relevanten Bereich repräsentativ ist, ohne Berücksichtigung der Intensität der Pixel jedes Ausschlussbereichs,

wobei das Verfahren auch ein Mischen der Probe mit einem zweiten Reagens, Lysereagens (26) genannt, umfasst, das geeignet ist, die Partikel zu lysieren, wobei jeder Ausschlussbereich der Spur (28) einer Luftblase (27) entspricht, wobei die Luftblase infolge der Lyse der Partikel gebildet wird.

2. Verfahren nach Anspruch 1, bei dem der Teilschritt ii) das Bestimmen mindestens eines Ausschlussbereichs (REX) umfasst, wobei der relevante Bereich (ROI) einem Teil der Messzone (ZM) entspricht, der jeden so bestimmten Ausschlussbereich nicht umfasst.

3. Verfahren nach einem der Ansprüche 1 oder 2, umfassend ferner, beim Schritt d),

    - das Bestimmen eines Lyseindikators (Ind(ti)) anhand des beim Schritt c) erfassten Bildes,
    - wobei das Schätzen der Analytmenge ausgeführt wird, wenn dieser Lyseindikator ein vorbestimmtes Lysekriterium erfüllt.

4. Verfahren nach Anspruch 3, bei dem, wenn der Lyseindikator das Lysekriterium nicht erfüllt, der Schritt c) wiederholt wird, so dass eine Mehrzahl von Lyseindikatoren (Ind(ti)) anhand von Bildern ($I(t_i)$, $I(t_{i-1})$), die zu verschiedenen Zeitpunkten ($t_i$, $t_{i-1}$) erfasst wurden, bestimmt wird.

5. Verfahren nach Anspruch 4, bei dem jeder Lyseindikator bestimmt wird, indem ein Vergleichsbild ($\Delta t_i$) gebildet wird, das einen Vergleich zwischen zwei Bildern ($I(t_i)$, ($I(t_{i-1})$) darstellt, die zu verschiedenen Zeitpunkten ($t_i$, $t_{i-1}$) erfasst wurden.

6. Verfahren nach einem der vorhergehenden Ansprüche, umfassend einen Schritt des Einführens der Probe in eine Fluidkammer (13), wobei die Schritte c) und d) ausgehend von einem vorbestimmten Zeitpunkt (T) nach dem Einführen durchgeführt werden.

7. Verfahren nach einem der vorhergehenden Ansprüche, umfassend das Erfassen einer Mehrzahl von aufeinanderfolgenden Bildern (I, I') der von der Probe durchgelassenen oder reflektierten Lichtstrahlung, wobei die Analytmenge beim Schritt d) in Abhängigkeit vom Verlauf der Intensität jedes Bildes in Abhängigkeit von der Zeit bestimmt wird.

8. Verfahren nach einem der vorhergehenden Ansprüche, bei dem beim Schritt d) die Größe (k), die für die Intensität der Pixel in dem relevanten Bereich (ROI) repräsentativ ist, das Integral oder den Mittelwert der Pixel in dem relevanten Bereich umfasst.

9. Verfahren nach einem der vorhergehenden Ansprüche, bei dem der Ausschlussbereich (REX) durch Schwellenwertbildung des Bildes (I, I') oder durch Segmentierung des Bildes (I, I') bestimmt wird.

10. Verfahren nach einem der vorhergehenden Ansprüche, bei dem die Partikel (20) rote Blutkörperchen sind oder der Analyt (30) Glucose ist.

11. Verfahren nach einem der vorhergehenden Ansprüche, bei dem der Matrix-Fotodetektor (16) in einem Abstand (d) von der Probe von weniger als 1 cm gelegen ist.

12. Verfahren nach einem der vorhergehenden Ansprüche, bei dem es keine Vergrößerungsoptik zwischen der Probe (10) und dem Matrix-Fotodetektor (16) gibt.

13. Verfahren nach einem der vorhergehenden Ansprüche, bei dem die Probe (10) zwischen der Lichtquelle (11) und dem Matrix-Fotodetektor (16) angeordnet ist, so dass der Matrix-Fotodetektor eine von der Probe (10) durchgelassene Strahlung (14) detektiert, wobei das von dem Matrix-Fotodetektor erfasste Bild dann ein sogenanntes Transmissionsbild (I) ist.

14. Verfahren nach einem der Ansprüche 1 bis 12, bei dem die Probe gegenüber der Lichtquelle (11) und dem Matrix-Fotodetektor (16) angeordnet ist, so dass der Matrix-Fotodetektor eine von der Probe (10) reflektierte oder rückgestreute Strahlung (14') detektiert, wobei das von dem Matrix-Fotodetektor erfasste Bild dann ein sogenanntes Reflexionsbild (I') ist.

**Claims**

1. Method for estimating an amount of an analyte (30) in a liquid sample (10), comprising particles (20), the method comprising the following steps:

    a) mixing said sample (10) with a first reagent (22) capable of forming a coloured indicator (24) in the presence of said analyte, in the sample,
    b) following this mixing, illuminating said sample (10) using a light source (11) capable of emitting light radiation (12) towards the sample,
    c) acquiring, using a matrix photodetector (16), an image (I, I') of light radiation (14,14') transmitted or reflected by the sample,
    d) estimating an amount of said analyte (30) as a function of said image, according to the following sub-steps:

        i) selecting a measurement zone (ZM), in said image, said measurement zone comprising a plurality of pixels,

    the method being **characterized in that** it also comprises:

        ii) determining, inside the measurement zone (ZM), a region of interest (ROI) and at least one region of exclusion (REX),
        iii) estimating said amount of analyte (30) from a quantity (k) representative of the intensity of the pixels in said region of interest, without taking into account the intensity of the pixels of each region of exclusion,

    the method also comprising a mixing of the sample with a second reagent, referred to as lysis reagent (26), capable of lysing said particles, each region of exclusion corresponding to the mark (28) of an air bubble (27), said air bubble being formed following the lysis of said particles.

2. Method according to Claim 1, in which sub-step ii) comprises determining at least one region of exclusion (REX), said region of interest (ROI) corresponding to a portion of said measurement zone (ZM) not including each region of exclusion thus determined.

3. Method according to either of Claims 1 and 2, also comprising, during step d),

    - determining a lysis indicator (Ind(ti)) from the image acquired during step c),
    - the amount of analyte being estimated when this lysis indicator satisfies a predetermined lysis criterion.

4. Method according to Claim 3, in which, when the lysis indicator does not satisfy said lysis criterion, step c) is repeated, so that a plurality of lysis indicators (Ind(ti)) are determined, from images ($I(t_i)$, ($I(t_{i-1})$) acquired at various times ($t_i$, $t_{i-1}$).

5. Method according to Claim 4, in which each lysis indicator is determined by forming a comparison image ($\Delta t_i$), representing a comparison between two images ($I(t_i)$, ($I(t_{i-1})$) acquired at various times ($t_i$, $t_{i-1}$).

6. Method according to any one of the preceding claims, comprising a step of introducing the sample into a fluidic chamber (13), steps c) and d) being carried out starting from a predetermined time (T) after said introduction.

7. Method according to any one of the preceding claims, comprising the acquisition of a plurality of successive images (I, I') of the light radiation transmitted or reflected by the sample, the amount of analyte being determined, during step d), as a function of the change in the intensity of each image as a function of time.

8. Method according to any one of the preceding claims, in which, during step d), said quantity (k) representative of the intensity of the pixels in said region of interest (ROI) comprises the integral or the mean of the pixels in said region of interest.

9. Method according to any one of the preceding claims, in which the region of exclusion (REX) is determined by thresholding said image (I, I'), or by segmenting said image (I, I').

10. Method according to any one of the preceding claims, in which said particles (20) are red blood cells, or that the analyte (30) is glucose.

11. Method according to any one of the preceding claims, in which the matrix photodetector (16) is located at a distance (d) from the sample of less than 1 cm.

12. Method according to any one of the preceding claims, in which there is no magnifying optic between the sample (10) and the matrix photodetector (16).

13. Method according to any one of the preceding claims, in which the sample (10) is positioned between the light source (11) and the matrix photodetector (16) so that the matrix photodetector detects radiation (14) transmitted by the sample (10), the image acquired by the matrix photodetector then being a so-called transmission image (I).

14. Method according to any one of Claims 1 to 12, in which the sample is positioned facing the light source (11) and the matrix photodetector (16), so that the matrix photodetector detects radiation (14') reflected or backscattered by the sample (10), the image acquired by the matrix photodetector then being a so-called reflection image (I').

**Fig. 1**

**Fig. 2**

**Fig. 3A**

**Fig. 3B**

**Fig. 4A**

**Fig. 4B**

**Fig. 5**

100

↓

200

↓

300

$I(t_i)$

↓

320

↓

340

$Ind(t_i)$

↓

360     100'

$I = I_{T_1}$

↓

400

ROI, REX     $I_o$

↓

500

k

↓

600

**Fig. 6**

**Fig. 7A**

**Fig. 7B**

**Fig. 7C**

**Fig. 7D**

**Fig. 7E**

**Fig. 7F**

**Fig. 8A**

**Fig. 8B**

**Fig. 8C**

**Fig. 9A**

**Fig. 9B**

**Fig. 9C**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5866349 A
- EP 1875203 A
- US 20150160244 A
- US 20130126712 A
- US 3964974 A